(19) Europäisches Patentamt
European Patent Office
Office européen des brevets

(11) Publication number: 0 054 909
A2

# (12) EUROPEAN PATENT APPLICATION

(21) Application number: **81110514.7**

(22) Date of filing: **16.12.81**

(51) Int. Cl.³: **C 07 C 59/56**, C 07 C 69/732, C 07 C 33/42, C 07 D 303/12, C 07 D 309/12, C 07 D 407/12

(30) Priority: **18.12.80 JP 179403/80**
**18.12.80 JP 179404/80**
**19.12.80 JP 180253/80**
**22.12.80 JP 181697/80**

(43) Date of publication of application: **30.06.82**
**Bulletin 82/26**

(84) Designated Contracting States: **AT BE CH DE FR GB IT LI NL SE**

(71) Applicant: **MITSUBISHI CHEMICAL INDUSTRIES LIMITED, 5-2, Marunouchi 2-chome Chiyoda-ku, Tokyo 100 (JP)**

(72) Inventor: **Takayanagi, Hisao, 23-4, Tana-cho Midori-ku, Yokohama-shi Kanagawa (JP)**
Inventor: **Morita, Yoshiharu, 1988-52, Kitahassaku-cho Midori-ku, Yokohama-shi Kanagawa (JP)**

(74) Representative: **Ter Meer-Müller-Steinmeister Patentanwälte, Triftstrasse 4, D-8000 München 22 (DE)**

## (54) 9-Halo-8-hydroxy-4-methyl-4-nonenoic acids, derivatives thereof and a process for their preparation.

(57) 9-Halo-8-hydroxy-4-methyl-4-nonenoic acids and derivatives thereof have been prepared, which are important intermediates for the full synthesis of (1RS, 4SR, 5RS)-4-(4,8-dimethyl-5-hydroxy-7-nonenyl)-4-methyl-3,8-dioxabicyclo[3.2.1]-octane-1-acetic acid [A]:

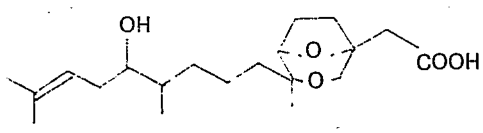

[A]

having high activity as an utero-evacuant agent.

EP 0 054 909 A2

# 9-HALO-8-HYDROXY-4-METHYL-4-NONENOIC ACIDS, DERIVATIVES THEREOF AND A PROCESS FOR THEIR PREPARATION

## BACKGROUND OF THE INVENTION

### Field of the Invention

This invention relates to 9-halo-8-hydroxy-4-methyl-4-nonenoic acids and derivatives thereof and a process for preparing the same. More particularly, it relates to 9-halo-8-hydroxy-4-methyl-4-nonenoic acids and derivatives thereof which are important intermediates for the full synthesis of (1RS, 4SR, 5RS)-4-(4,8-dimethyl-5-hydroxy-7-nonenyl)-4-methyl-3,8-dioxabicyclo[3.2.1]-octane-1-acetic acid [A]:

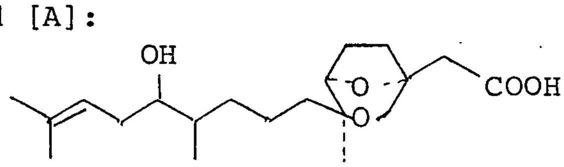

[A]

having high activity as an utero-evacuant agent (see U.S. Patent Nos. 4,102,895 and 4,215,048), and a process for preparing the same.

## SUMMARY OF THE INVENTION

Upon our earnest study on the full synthesis of (1RS, 4SR, 5RS)-4-(4,8-dimethyl-5-hydroxy-7-nonenyl)-4-methyl-3,8-dioxabicyclo-[3.2.1]octane-1-acetic acid, we have found that 9-halo-8-hydroxy-4-methyl-4-nonenoic acids and their derivatives are useful intermediates for this purpose and accomplished the present invention.

Thus, in brief, the present invention resides, in one aspect, in a 9-halo-8-hydroxy-4-methyl-4-nonenoic acids and their derivatives of the formula:

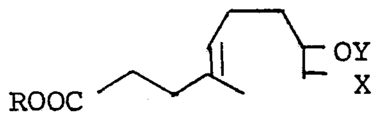
(I)

wherein X is a halogen atom, Y is hydrogen or a hydroxy-protecting group, and R is hydrogen or a lower alkyl.

These compounds may be prepared by reacting a prenyl halide of the formula:

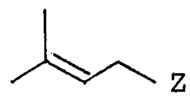
(II)

wherein Z is a halogen atom,

with magnesium to give a prenyl magnesium halide, then reacting the resulting compound with an epihalohydrin of the formula:

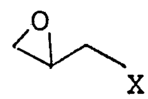
(III)

wherein X is as defined above,

and, if desired, protecting the hydroxyl group of the product with a protecting agent to give a 1-halo-6-methyl-5-hepten-2-ol or its derivative of the formula:

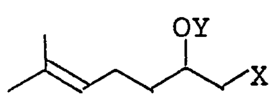
(IV)

wherein X and Y are as defined above,

then epoxidizing the resulting compound to give a 1-halo-5,6-epoxy-6-methyl-2-heptanol or its derivative of the formula:

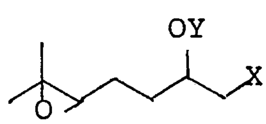
(V)

wherein X and Y are as defined above,

subjecting the resulting epoxy compound to rearrangement to give a 1-halo-5-hydroxy-6-methylene-2-heptanol or its derivative of the formula:

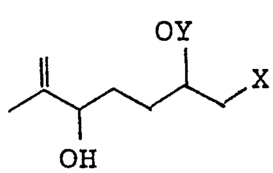
(VI)

wherein X and Y are as defined above,

reacting the rearranged product with an orthoacetic acid ester

of the formula:

$$CH_3C(OR')_3 \qquad (VII)$$

wherein R' is a lower alkyl,

in the presence of a weak acid, followed by hydrolysis, to give the desired 9-halo-8-hydroxy-4-methyl-4-nonenoic acid or its derivative.

## DESCRIPTION OF THE PREFERRED EMBODIMENTS

As mentioned above, in Formula (I), X represents a halogen atom, Y is hydrogen or a hydroxy-protecting group and R is hydrogen or a lower alkyl having 1 to 4 carbon atoms. Examples of the hydroxy-protecting group for Y include: 2-tetrahydropyranyl, 1-methoxymethyl, 1-methoxyethyl, 1-ethoxyethyl, 1-ethoxy-n-propyl, 1-n-butyloxyethyl, 1-isobutyloxyethyl, 1-methoxy-1-methylethyl, 1-ethoxy-1-methylethyl, benzyl, trimethylsilyl.

The 9-halo-8-hydroxy-4-methyl-4-nonenoic acids and their derivatives includes, for example:

9-bromo-8-hydroxy-4-methyl-4-nonenoic acid;
9-chloro-8-hydroxy-4-methyl-4-nonenoic acid;
1-bromo-6-carbomethoxyethyl-5-hepten-2-yl-tetrahydropyran-2-ylether;
1-chloro-6-carbomethoxyethyl-5-hepten-2-yl-tetrahydropyran-2-ylether;
1-bromo-6-carbethoxyethyl-5-hepten-2-yl-tetrahydropyran-2-ylether;
1-chloro-6-carbethoxyethyl-5-hepten-2-yl-tetrahydropyran-2-ylether;
1-bromo-6-carbobutoxyethyl-5-hepten-2-yl-tetrahydropyran-2-ylether;
1-chloro-6-carbobutoxyethyl-5-hepten-2-yl-tetrahydropyran-2-ylether;
1-bromo-6-carbethoxyethyl-5-hepten-2-yl-1-methoxyethylether;
1-chloro-6-carbethoxyethyl-5-hepten-2-yl-1-methoxyethylether;
1-bromo-6-carbomethoxyethyl-5-hepten-2-yl-1-methoxyethylether;
1-chloro-6-carbomethoxyethyl-5-hepten-2-yl-1-methoxyethylether;
1-bromo-6-carbethoxyethyl-5-hepten-2-yl-(1-n-butoxyethyl)ether;
1-chloro-6-carbethoxyethyl-5-hepten-2-yl-(1-n-butoxyethyl)ether;
1-bromo-6-carbomethoxyethyl-5-hepten-2-yl-(1-n-butoxyethyl)ether;
1-chloro-6-carbomethoxyethyl-5-hepten-2-yl-(1-n-butoxyethyl)ether;
1-bromo-6-carbethoxyethyl-5-hepten-2-yl-(1-isobutyloxyethyl)ether;
1-chloro-6-carbethoxyethyl-5-hepten-2-yl-(1-isobutyloxyethyl)ether;
1-bromo-6-carbomethoxyethyl-5-hepten-2-yl-(1-isobutyloxyethyl)ether;

1-chloro-6-carbomethoxyethyl-5-hepten-2-yl-(1-isobutyloxyethyl) ether;
1-chloro-6-carbethoxyethyl-5-hepten-2-yl-benzyl-ether;
1-chloro-6-carbethoxyethyl-5-hepten-2-yl-trimethylsilyl-ether; and
1-iodo-6-carbethoxyethyl-5-hepten-2-yl-trimethylsilyl-ether.

The compounds according to this invention are useful as intermediates from which (1RS, 4SR, 5RS)-4-(4,8-dimethyl-5-hydroxy-7-nonenyl)-4-methyl-3,8-dioxabicyclo[3.2.1]octane-1-acetic acid [A] which is useful as an utero-evacuant agent can be derived by the following reaction sequence, for example:

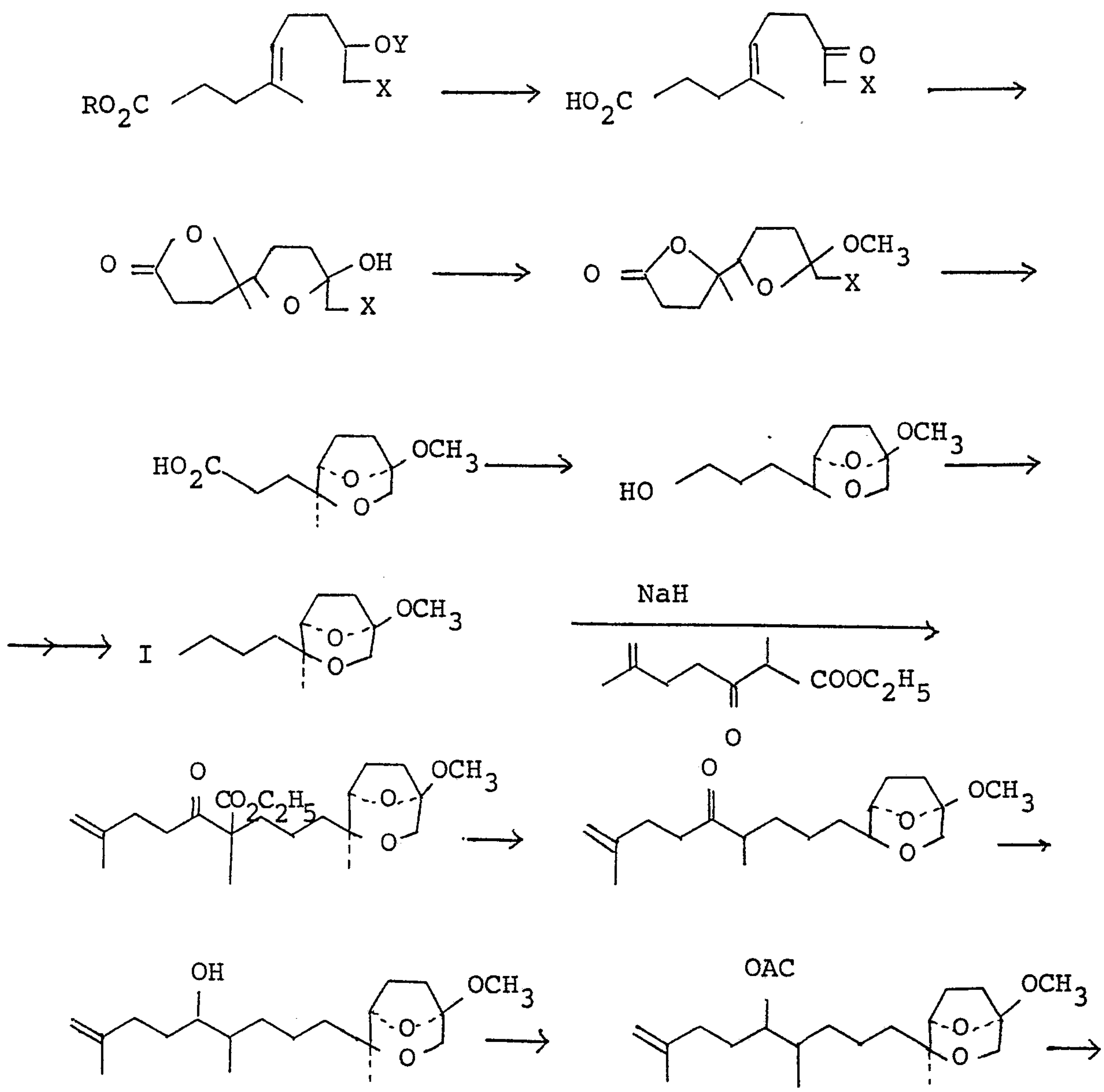

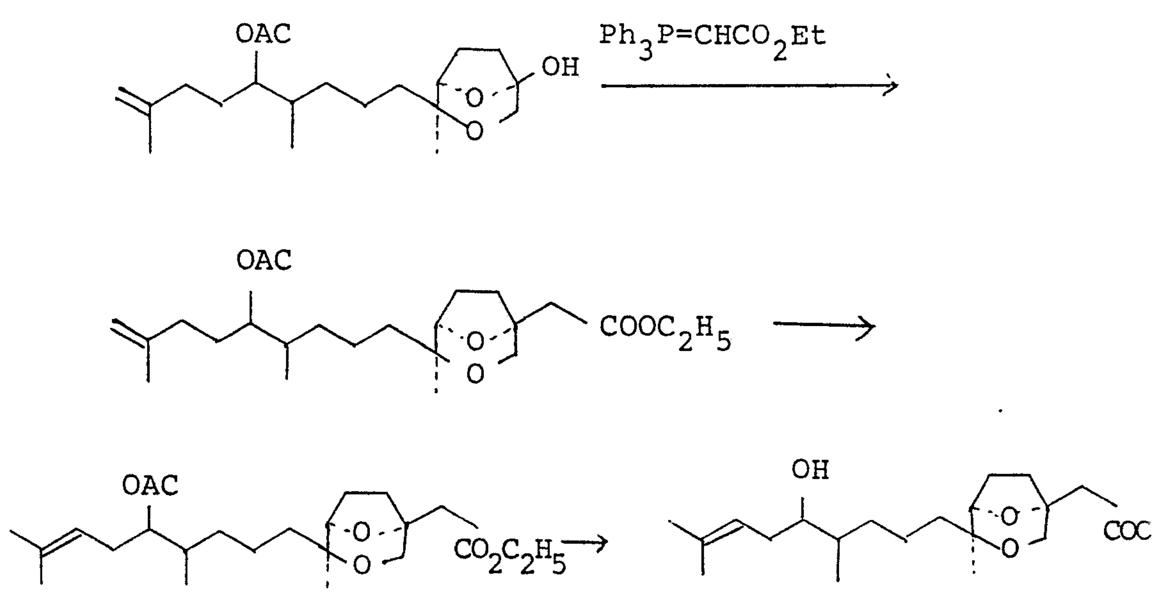


The above reaction sequence provides an advantageous process for the commercial preparation of Compound [A] and the compounds according to this invention are therefore valuable and important intermediates for the synthesis of Compound [A].

The compounds of the present invention may be prepared, for example, by the aforementioned process, which will be described below more fully in sequence of reactions.

(1) Preparation of 1-halo-6-methyl-5-hepten-2-ols and their derivatives (IV)

A prenyl halide (II), one of the starting materials, is reacted with magnesium in a conventional manner to give a prenyl magnesium halide. The reaction is carried out usually in an etheric solvent such as ether, tetrahydrofuran, dimethoxyethane or the like at a temperature of from -30°C to room temperature, preferably from -20 to 0°C. Magnesium is used in an equimolar or small excess amount based on the prenyl halide. In order to allow the reaction to proceed smoothly, a small amount of iodine, an alkyl bromide, an alkylene dibromide or the like may be added to activate the magnesium. The reaction is completed usually in 20 minutes to several hours.

The resulting prenyl magnesium halide is then reacted with an epihalohydrin, which is normally used in solution in an etheric solvent exemplified above.

The reaction is preferably carried out by dropwise addition of a solution of prenyl magnesium halide to a solution of epihalohydrin, because such addition manner aids in improving the

selectivity of the reaction and minimizing the formation of byproducts. The reaction temperature is between -40°C and +50°C, preferably -30°C and 0°C. The epihalohydrin is used in an amount almost equimolar with the prenyl magnesium halide. The addition of a small amount of a cuprous halide such as cuprous iodide or cuprous bromide aids in improving the selectivity of the reaction. The cuprous halide may be added in an amount of 0.01 to 0.1 equivalent based on the epihalohydrin. The reaction is usually completed in 20 minutes to several hours. Thereafter, the reaction mixture may be worked up according to the conventional techniques in organic chemistry, for example, by acid-treatment, evaporation of solvent, washing, solvent extraction and distillation, to recover a purified product. However, even a product of low purity may successfully be used for aforementioned purposes (i.e., as a starting material in the subsequent step).

Preferably the thus obtained 1-halo-6-methyl-5-hepten-2-ol is then protected at the 2-hydroxyl group prior to use in the subsequent reaction when it is used as an intermediate for the synthesis of (1RS, 4SR, 5RS)-4-(4,8-dimethyl-5-hydroxy-7-nonenyl)-4-methyl-3,8-dioxabicyclo[3.2.1]octane-1-acetic acid [A]. The protection may be effected with a compound having the formula:

$$R^1 - O - C(R^2) = CHR^3 \qquad \text{(VIII) or}$$

$$R^4 X \qquad \text{(IX)}$$

wherein $R^1$ is a lower alkyl having 1 to 6 carbon atoms such as methyl, ethyl, propyl, butyl or hexyl;
$R^2$ is hydrogen or a lower alkyl having 1 to 4 carbon atoms such as methyl or ethyl;
$R^3$ is hydrogen or a lower alkyl having 1 to 6 carbon atoms such as methyl, ethyl, propyl, butyl or hexyl; or
$R^1$ and $R^3$ may together form an alkylene chain such as trimethylene, tetramethylene or pentamethylene;
$R^4$ is $-CH_2OCH_3$, $-CH_2OCH_2CH_2OCH_3$, $-CH_2Ph$ or $-Si(CH_3)_3$, and X is as defined above.

The compound of Formula (VIII) or (IX) includes, for example, 5,6-dihydro-4H-pyran, methyl vinyl ether, methyl isopropenyl ether, butyl vinyl ether, butyl isopropenyl ether, ethyl vinyl ether, chloromethyl methyl ether, chloromethyl propyl ether, benzyl chloride, benzyl bromide and trimethylsilyl chloride.

Among these protecting agents, alkyl vinyl ethers (VIII) are generally used in the presence of an acid catalyst to protect the hydroxyl group of a 1-halo-6-methyl-5-hepten-2-ol. Useful acid catalysts include p-toluenesulfonic acid, benzenesulfonic acid, methanesulfonic acid, trifluoromethanesulfonic acid, acetic acid, hydrogen chloride, phosphorus oxychloride, acidic ionexchange resin and the like. A solvent such as benzene, toluene, ether, tetrahydrofuran, chloroform, dichloromethane, ethyl acetate, dioxane, dimethylformamide or hexane may be used, although the reaction may be carried out without solvent. The reaction temperature is usually from room temperature to 80°C and the reaction time is from 10 minutes to several hours. The protecting agent of Formula (VIII) may usually be employed in an equimolar or small excess amount based on the amount of 1-halo-6-methyl-5-hepten-2-ol being protected. After the reaction, the reaction mixture is neutralized and the solvent and the excess protecting agent are distilled off to recover an etheric compound of the formula:

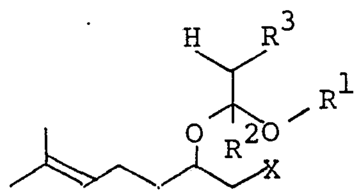

(X)

wherein $R^1$, $R^2$, $R^3$ and X are as defined above, said compound being the desired hydroxyl-protected 1-halo-6-methyl-5-hepten-2-ol.

On the other hand, when a halide (IX) is used as a protecting agent, a 1-halo-6-methyl-5-hepten-2-ol may be reacted with the halide in the presence of a base to achieve the purpose of protecting the hydroxyl group. The base which is used in the reaction includes tertiary amines such as triethylamine and pyridine, metal hydrides such as lithium hydride and sodium hydride and metal hydroxides such as sodium hydroxide and potassium hydroxide. The reaction is usually carried out by adding a base to a mixture of a 1-halo-6-methyl-5-hepten-2-ol and an equimolar or excess protecting agent dissolved in a suitable solvent (tetrahydrofuran, dimethylformamide, benzene, toluene, dioxane or the like). When an amine is used, the reaction may be conducted without solvent. The reaction temperature is usually from room temperature to 100°C and the reaction time is from 1 hour to 1 day.

Alternatively, in the preparation of 1-halo-6-methyl-5-hepten-2-ol, the resulting reaction mixture may be reacted with an equimolar or small excess halide (IX) at a temperature of from room temperature to refluxing temperature for 1 hour to 1 day, instead of the acid treatment, thereby directly yielding the desired hydroxyl-protected 1-halo-6-methyl-5-hepten-2-ol. In either case, the desired protected compound of the formula:

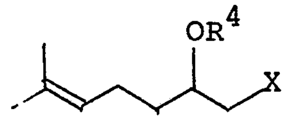

(XI)

wherein X and $R^4$ are as defined above,

may be isolated by subjecting the reaction mixture to washing, extraction and removal of solvent by distillation, for example.

The crude compound (X) or (XI) obtained as above may further be purified by column chromatography or the like in a conventional manner, while it is also possible to use the crude product in the subsequent step as it is without further purification.

The 1-halo-6-methyl-5-hepten-2-ols and their derivatives of Formulas (IV), (X) or (XI) include, for example:

1-bromo-6-methyl-5-hepten-2-ol;
1-chloro-6-methyl-5-hepten-2-ol;
1-iodo-6-methyl-5-hepten-2-ol;
1-bromo-6-methyl-5-hepten-2-yl-tetrahydropyran-2-ylehter;
1-chloro-6-methyl-5-hepten-2-yl-tetrahydropyran-2-ylether;
1-iodo-6-methyl-5-hepten-2-yl-tetrahydropyran-2-ylether;
1-bromo-6-methyl-5-hepten-2-yl-(1-methoxyethyl)-ether;
1-chloro-6-methyl-5-hepten-2-yl-(1-methoxyethyl)-ether;
1-chloro-6-methyl-5-hepten-2-yl-(1-ethoxyethyl)-ether;
1-bromo-6-methyl-5-hepten-2-yl-(1-ethoxyethyl)-ether;
1-chloro-6-methyl-5-hepten-2-yl-(1-n-butoxyethyl)-ether;
1-bromo-6-methyl-5-hepten-2-yl-(1-n-butoxyethyl)-ether;
1-chloro-6-methyl-5-hepten-2-yl-(1-isobutoxyethyl)-ether;
1-bromo-6-methyl-5-hepten-2-yl-(1-isobutoxyethyl)-ether;
1-chloro-6-methyl-5-hepten-2-yl-(methoxymethyl)-ether;
1-bromo-6-methyl-5-hepten-2-yl-(methoxymethyl)-ether;
1-chloro-6-methyl-5-hepten-2-yl-(2-methoxyethoxyethyl)-ether;
1-bromo-6-methyl-5-hepten-2-yl-(2-methoxyethoxyethyl)-ether;
1-chloro-6-methyl-5-hepten-2-yl-benzyl-ether;
1-bromo-6-methyl-5-hepten-2-yl-benzyl-ether;
1-chloro-6-methyl-5-hepten-2-yl-trimethylsilyl-ether; and

1-bromo-6-methyl-5-hepten-2-yl-trimethylsilyl-ether.

(2) Preparation of 1-halo-5,6-epoxy-6-methyl-2-heptanols and their derivatives (V)

The compound (V) may be prepared by epoxidization of a 1-halo-6-methyl-5-hepten-2-ol or its derivative obtained by the reaction(s) described in (1) above. The epoxidizing agent used in this reaction includes organic peracids such as m-chloroperbenzoic acid, perbenzoic acid and peracetic acid, organic hydroperoxides such as tert-butyl hydroperoxide, and hydrogen peroxide. When Y represents a protecting group, the desired epoxy compound (V) may also be prepared by reacting the hydroxyl-protected compound (IV) with a halogenating agent such as N-bromosuccinimide, N-chlorosuccinimide or t-butyl hypochlorite and then treating the resulting halohydrin compound with a base.

A wide variety of solvents including chloroform, dichloromethane, benzene and water may be used. The reaction is usually carried out at a temperature of -20°C to room temperature for 10 minutes to several hours.

The epoxidizing agent is used in an equimolar or slight excess amount based on the 1-halo-6-methyl-5-hepten-2-ol or its derivative (IV).

The reaction mixture may be worked up according to the conventional manner in organic chemistry, for example, by decomposition of the excess epoxidizing agent, washing, extraction and evaporation of solvent to isolate the desired product. The thus obtained crude product may further be purified by column chromatography or similar technique, while it can be used in the subsequent step as it is.

The 1-halo-5,6-epoxy-6-methyl-2-heptanols and their derivatives of Formula (V) include:

1-bromo-5,6-epoxy-6-methyl-2-heptanol;
1-chloro-5,6-epoxy-6-methyl-2-heptanol;
1-iodo-5,6-epoxy-6-methyl-2-heptanol;
1-bromo-5,6-epoxy-6-methyl-2-heptyl-tetrahydropyran-2-ylether;
1-chloro-5,6-epoxy-6-methyl-2-heptyl-tetrahydropyran-2-ylether;
1-iodo-5,6-epoxy-6-methyl-2-heptyl-tetrahydropyran-2-ylether;
1-bromo-5,6-epoxy-6-methyl-2-heptyl-(1-methoxyethyl)-ether;
1-chloro-5,6-epoxy-6-methyl-2-heptyl-(1-methoxyethyl)-ether;
1-iodo-5,6-epoxy-6-methyl-2-heptyl-(1-methoxyethyl)-ether;
1-chloro-5,6-epoxy-6-methyl-2-heptyl-benzyl-ether;

1-chloro-5,6-epoxy-6-methyl-2-heptyl-methoxymethyl-ether;
1-chloro-5,6-epoxy-6-methyl-2-heptyl-trimethylsilylether; and
1-bromo-5,6-epoxy-6-methyl-2-heptyl-benzyl-ether.

(3) Preparation of 1-halo-5-hydroxy-6-methylene-2-heptanols and their derivatives (VI)

The desired compound (VI) may be prepared by rearrangement of a 1-halo-5,6-epoxy-6-methyl-2-heptanol obtained in the reaction described in (2) above.

The rearrangement of an epoxy compound into an allyl alcohol may be performed, for example, by treatment with a dialkylboryltrifluoromethanesulfonate-tertiary amine [Chemistry Letters (1977) p.p. 1215 - 1218] or by reaction with an organosilicon compound such as trimethylsilyl trifluoromethanesulfonate [J. Am. Chem. Soc., 101, 2738-9 (1979)].

According to another simpler method described in Japanese Patent Laid-Open Specification No. 76507/1979, the epoxy (V) may also be treated with an aluminum trialkoxide of the formula:

$$Al(OR)_3$$

wherein R is a primary, secondary or tertiary lower alkoxy,

to effect the rearrangement. The aluminum trialkoxide useful in this method includes aluminum triisopropoxide and the like and it is generally used in an equimolar or small excess amount based on the epoxy compound (V). The reaction is carried out in a solvent such as toluene, benzene, xylene or the like at a temperature of 50 to 120°C for about 1 to 10 hours.

The resulting reaction mixture may be worked up, for example, by neutralization, extraction, washing and removal of solvent by distillation, according to the conventional techniques in the field of organic chemistry to isolate the desired compound (VI).

While the thus obtained crude compound (VI) may further be purified by chromatography or similar procedure, it is also possible to use the crude product in the subsequent step as it is.

The 1-halo-5-hydroxy-6-methylene-2-heptanol or its derivative of Formula (VI) includes, for example:

1-bromo-5-hydroxy-6-methylene-2-heptanol;
1-chloro-5-hydroxy-6-methylene-2-heptanol;
1-iodo-5-hydroxy-6-methylene-2-heptanol;
1-bromo-5-hydroxy-6-methylene-2-heptyl-tetrahydropyran-2-ylether;
1-chloro-5-hydroxy-6-methylene-2-heptyl-tetrahydropyran-2-yl-

ether;
1-iodo-5-hydroxy-6-methylene-2-heptyl-tetrahydropyran-2-ylether;
1-bromo-5-hydroxy-6-methylene-2-heptyl-(1-methoxyethyl)-ether;
1-chloro-5-hydroxy-6-methylene-2-heptyl-(1-methoxyethyl)-ether;
1-iodo-5-hydroxy-6-methylene-2-heptyl-(1-methoxyethyl)-ether;
1-bromo-5-hydroxy-6-methylene-2-heptyl-(1-n-butoxyethyl)-ether;
1-chloro-5-hydroxy-6-methylene-2-heptyl-(1-n-butoxyethyl)-ether;
1-bromo-5-hydroxy-6-methylene-2-heptyl-(1-isobutoxyethyl)-ether;
1-chloro-5-hydroxy-6-methylene-2-heptyl-(1-isobutoxyethyl)-ether;
1-chloro-5-hydroxy-6-methylene-2-heptyl-methoxymethyl-ether;
1-chloro-5-hydroxy-6-methyl-2-heptyl-benzyl-ether;
1-chloro-5-hydroxy-6-methyl-2-heptyl-trimethylsilyl-ether;
1-bromo-5-hydroxy-6-methylene-2-heptyl-methoxymethyl-ether; and
1-iodo-5-hydroxy-6-methyl-2-heptyl-benzyl-ether.

(4) Preparation of 9-halo-8-hydroxy-4-methyl-4-nonenoic acids and their derivatives (I)

The compounds (I) may be prepared by reacting a 1-halo-2-(substituted oxy)-5-hydroxy-6-methylene-heptane (VI) with an orthoacetic acid ester of Formula (VII) in the presence of a weak acid such as propionic acid, butyric acid or the like as a catalyst. Based on the compound (VI), the orthoacetic acid ester (VII) is used in an equimolar to large excess amount and an amount of 0.01 to 0.1 equivalent of the weak acid is adequate as the catalyst. The excess orthoacetic acid ester (VII) may also serve as the solvent, or any suitable solvent such as xylene may be employed. The reaction may usually be carried out at a temperature of about 120 to 150°C and satisfactory results are obtained when the reaction is allowed to proceed with distilling off the alcohol formed by the reaction. The reaction is usually completed in 20 minutes to several hours. The resulting reaction mixture may be worked up by removal of solvent and/or excess orthoacetic acid ester, extraction, washing and removal of solvent to isolate a 9-halo-8-(substituted oxy)-4-methyl-4-nonenoic acid ester of the formula:

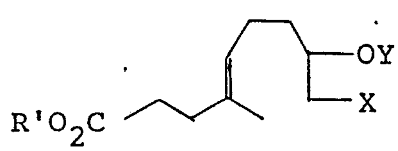

(XII)

wherein X, Y and R' are as defined above.

The crude ester (XII) thus obtained may further be purified by column chromatography or similar procedure. However, it is also

possible to employ the crude ester in the subsequent hydrolysis as it is.

The 9-halo-8-(substituted oxy)-4-methyl-4-nonenoic acid ester (XII) obtained as above is then subjected to acidic or basic hydrolysis or a combination of these whereby one of the ester moiety and the protected hydroxyl group or both of these are hydrolyzed to yield a product of the formula:

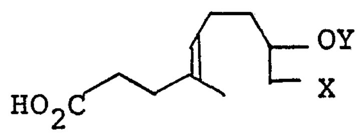

(I-a)

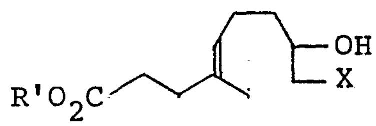

(I-b) or

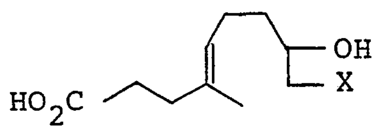

(I-c)

wherein X, Y and R' are as defined above.

The hydrolysis may be carried out in a conventional manner in the yield of organic chemistry using an acid such as hydrochloric acid, sulfuric acid or p-toluenesulfonic acid, or a base such as sodium hydroxide or potassium hydroxide. For example, hydrolysis of a compound (XII) with an alkali causes hydrolysis of only the ester moiety, resulting in the formation of a free carboxylic acid of Formula (I-a), while an acidic hydrolysis causes only the elimination of the hydroxyl-protecting group to give an alcoholic product of Formula (I-b). A 9-halo-8-hydroxy-4-methyl-4-nonenoic acid of Formula (I-c) may be obtained by subjecting a free carboxylic acid (I-a) prepared as above to further acidic hydrolysis. The compound (I-a), (I-b) or (I-c) thus obtained may be isolated and purified in a conventional manner in organic chemistry.

Having generally described the invention, a more complete understanding can be obtained by reference to certain specific examples, which are included for purposes of illustration only and are not intended to be limiting unless otherwise specified.

Example 1

(A) 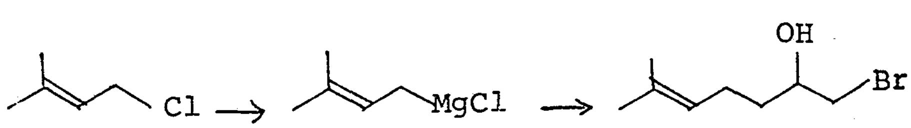

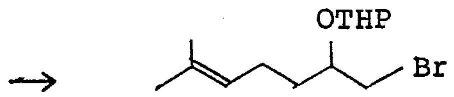

Magnesium (670 mg, 27.6 mmole) was added to tetrahydrofuran (30 ml) and the mixture was cooled to -10°C. A solution of prenyl chloride (2.6 g, 25 mmole) in tetrahydrofuran (15 ml) was then added dropwise under stirring at such a rate that the reaction temperature was kept at -5°C or below. The mixture was allowed to react for 30 minutes under stirring and then added dropwise to a stirred mixture of epibromohydrin (3.4 g, 25 mmole) and cuprous bromide (358 mg, 2.5 mmole) in tetrahydrofuran (10 ml) at such a rate that the reaction temperature was kept at -10°C or below. After stirring for 30 minutes, glacial acetic acid (1.5 g, 25 mmole) was added and stirring was continued for an additional 30 minutes. The solvent was then distilled off at reduced pressure. To the residue were added aqueous saturated ammonium chloride solution (20 ml) and water (20 ml) and the mixture was stirred for 4 hours. Ethyl ether (50 ml) was then added to extract the product thereinto. The organic layer was washed with water, dried and concentrated to give crude 1-bromo-6-methyl-5-hepten-2-ol (5.05 g, 57% purity). The crude product was dissolved in ethyl ether (10 ml) and 2,3-dihydropyran (2.6 g) was also dissolved therein. After the resulting solution was cooled on an ice-water bath, p-toluenesulfonic acid (about 10 mg) was added and stirred, thereby initiating an exothermic reaction and raising the temperature to about 25°C. The starting material disappeared after 30 minutes when the exotherm abated. Thereafter saturated sodium bicarbonate solution (10 ml) was added and the organic layer was separated. The organic layer was washed with water and saturated saline, dried ($MgSO_4$) and concentrated. The residue (7.53 g) was subjected to chromatography on silica gel and eluted with hexane-ethyl acetate (15:1). The appropriate fractions were collected and concentrated to give the desired tetrahydropyranyl ether derivative (1-bromo-6-methyl-5-hepten-2-yl-tetrahydropyran-2-ylether, 3.4 g, 47%).

IR(film)$cm^{-1}$: 2950, 2870, 1440, 1200, 1120, 1075, 1025, 975.

PMR ($CDCl_3$, 100 MHz) $\delta$ : 1.63 and 1.71 (each s, each 3H, $CH_3$ x 2), 3.25-4.10 (m, 5H, $HOC\underline{H}C\underline{H}_2Br$, $-CH_2O-$), 4.63 and 4.75 (bt, total 1H, J=3.5 Hz, $-C\underline{H}O-$), 5.09 (m, 1H, $C\underline{H}=C<$).

(B) 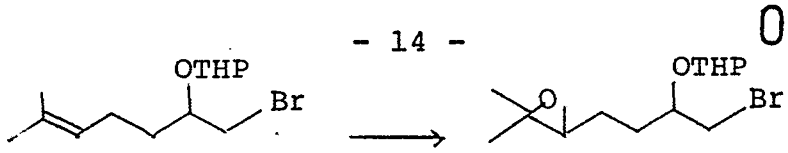

The olefin compound (1-bromo-6-methyl-5-hepten-2-yl-tetrahydropyran-2-yl-ether, 2.84 g, 9.8 mmole) was dissolved in methylene chloride (50 ml) and sodium bicarbonate (1.65 g, 19.6 mmole) was added thereto. The mixture was cooled to 0°C and a solution of m-chloroperbenzoic acid (m-CPBA) (80% purity, 2.21 g, 10.3 mmole) in methylene chloride (20 ml) was added dropwise. After disappearance of the starting material had been confirmed, saturated sodium sulfite solution (10 ml) and water (20 ml) were added and stirred for 1 hour. The organic layer was then separated, washed with water and saturated saline, dried ($MgSO_4$) and concentrated. The residue was subjected to chromatography on silica gel column and eluted with n-hexane-ethyl acetate (5:1) and the appropriate fractions were collected and concentrated to give 1-bromo-5,6-epoxy-6-methyl-2-heptyl-tetrahydropyran-2-ylether (2.85 g, 95%).

IR (film)$cm^{-1}$: 2950, 2870, 1380, 1200, 1125, 1075, 1035, 1025, 975.

PMR ($CDCl_3$, 100 MHz)$\delta$ ppm: 1.29 and 1.32 (each s, each 3H, $\underline{CH_3}$x2), 2.78 (m, 1H,

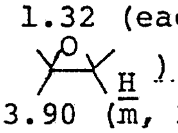

), 3.35-3.65 (m, 3H, -OC$\underline{H}$C$\underline{H_2}$Br), 3.90 (m, 2H, -C$\underline{H_2}$O-), 4.72 (m, 1H, -OC$\underline{H}$O-).

(C) 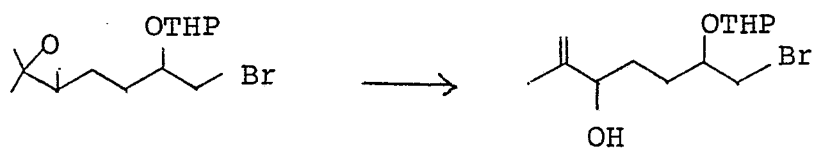

The epoxy compound (1-bromo-5,6-epoxy-6-methyl-2-heptyl-tetrahydropyran-2-yl-ether, 2.50 g, 8.17 mmole) was dissolved in toluene (14 ml) and aluminum isopropoxide (1.65 g, 8.1 mmole) was added to the solution. The mixture was allowed to react by heating at reflux for 5 hours. After cooling of the reaction mixture, 1N hydrochloric acid (8.1 ml) was added and stirred for 1 hour and the organic layer was separated. The aqueous layer was extracted three times with ethyl ether (10 ml) and the combined etheric extracts and organic layer obtained above were washed successively with water and saturated sodium bicarbonate solution and then dried and concentrated. The residue (2.5 g) was subjected to chromatography on silica gel column and eluted with n-hexane-ethyl acetate (4:1) to give the desired allyl alcohol compound (1-bromo-5-hydroxy-6-methylene-2-heptyl-tetrahydropyran-2-yl-

ether, 2.38 g, 95%).

IR (film) $cm^{-1}$: 3300, 3070, 2950, 2860, 1645, 1440, 1135, 1080, 1025, 980, 905, 870.

PMR ($CDCl_3$, 100 MHz) δ ppm : 1.74 (s, 3H, $CH_3$), 3.3-3.6 (m, 3H, $-OCHCH_2Br$), 3.85 (m, 2H, $-OCH_2-$), 4.06 (m, 1H, -CHOH), 4.70 (m, 1H, -OCHO-), 4.83 and 4.93 (bs, each 1H, $CH_2=C<$).

(D) 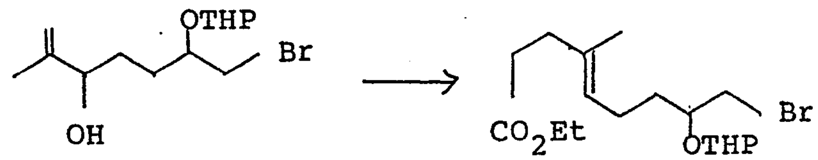

The allyl alcohol compound (1-bromo-5-hydroxy-6-methylene-2-heptyl-tetrahydropyran-2-yl-ether, 340 mg, 1.11 mmole) was dissolved in triethyl orthoacetate (1.0 g, 6.4 mmole) and propionic acid (5 mg) was added. The mixture was allowed to react by heating on a bath at 140°C while the ethanol formed by the reaction was distilled off. After 1 hour when the starting material had disappeared, the excess triethyl orthoacetate and propionic acid were distilled off at reduced pressure and the residue was purified by subjecting to chromatography on silica gel column and eluting with n-hexane-ethyl acetate (4:1) to give the desired ester (1-bromo-6-carbethoxyethyl-5-hepten-2-yl-tetrahydropyran-2-yl-ether, 350 mg, 84%).

IR (film) $cm^{-1}$ : 2940, 2860, 1730, 1440, 1370, 1240, 1075, 1020, 990, 870.

PMR ($CDCl_3$, 100 MHz) δ ppm: 1.25 (t, 3H, J=7.5 Hz, $CH_3CH_2-$), 1.63 (s, 3H, $CH_3$), 3.20-4.00 (m, 5H, $-OCHCH_2Br$, $-CH_2O-$), 4.10 (q, 2H, J=7.5 Hz, $CH_3CH_2-$), 4.60 and 4.73 (bs, total 1H, -OCHO-), 5.15 (bt, 1H, J=6.5 Hz, $CH=C<$).

(E) 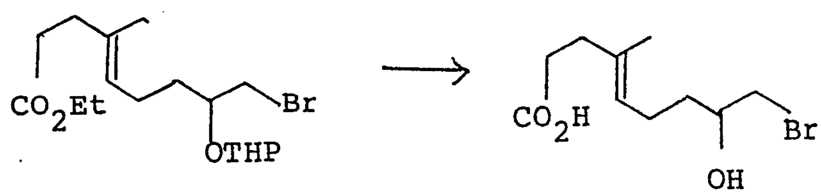

The pyranyl ether (ester) compound (1-bromo-6-carbethoxyethyl-5-hepten-2-yl-tetrahydropyran-2-yl-ether, 1.4 g, 4.0 mmole) was dissolved in a mixture of tetrahydrofuran (15 ml) and water (6 ml) and 2N hydrochloric acid (2 ml) was then added. The mixture was allowed to stand overnight at room temperature. Thereafter

most of the organic solvent was distilled off at reduced pressure and the organics in the residue were extracted into ether. The separated ether layer was washed with water, dried and concentrated and the resulting residue was purified by chromatography on silica gel column and eluting with n-hexane-ethyl acetate (1:1) to give the desired bromohydrin compound (9-bromo-8-hydroxy-4-methyl-4-nonenoic acid, 900 mg, 85%).

IR (film) $cm^{-1}$ : 3350, 2930, 2850, 1705, 1440, 1260, 1020, 860.

PMR ($CDCl_3$, 100 MHz)$\delta$ ppm: 1.64 (s, 3H, $CH_3$-), 3.39 (dd, 1H, J=13 and 6.5 Hz, -CHaHbBr), 3.48 (dd, 1H, J=13 and 4 Hz, -CHaHbBr), 3.76 (m, 1H, -CHOH), 5.15 (bt, 1H, J=6.5 Hz, CH=C<), 6.68 (bs, 1H, OH).

Example 2

(A)

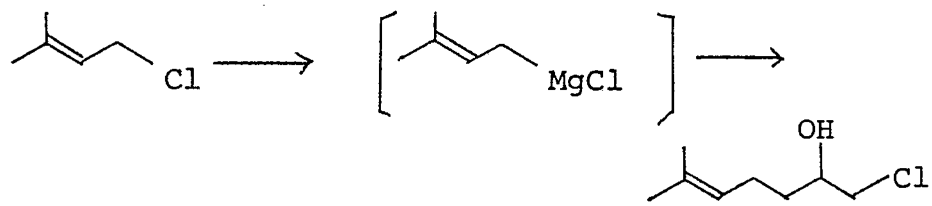

Magnesium (7.7 g, 0.32 mole) was added to tetrahydrofuran (300 ml) and iodine (50 mg) and dibromoethane (0.5 ml) were added then and stirred. After the color of iodine had disappeared, the mixture was cooled to -10°C and a solution of prenyl chloride (30 g, 0.287 mole) in tetrahydrofuran (150 ml) was added dropwise under stirring. The rate of addition was adjusted so as to maintain the reaction temperature at -5°C or below. After stirring for 30 minutes to allow the reaction to proceed, the resulting mixture was added dropwise to a mixture of epichlorohydrin (26.5 g, 0.287 mole) and cuprous iodide (5.5 g, 28.9 mmole) in tetrahydrofuran (100 ml) under stirring at such a rate that the reaction temperature was kept at -10°C or below. After stirring for another 30 minutes, acetic acid (17.5 ml, 0.29 mole) was added and stirring was continued for an additional 30 minutes. The solvent was then distilled off at reduced pressure and to the residue were added saturated ammonium chloride solution (200 ml) and water (100 ml) and stirred for 3 hours. The organic layer was separated and the aqueous layer was extracted three times each with ethyl acetate (100 ml). The ethyl acetate layers were combined with the organic layer obtained above and washed with water, dried and concentrated.

The residue was distilled in vacuo to give 1-chloro-6-methyl-5-hepten-2-ol (36.2 g, 83% purity, 65% net yield), b.p. 65 - 68°C/ 2 mmHg.

IR (film) $cm^{-1}$ : 3350, 2960, 2920, 2850, 1670, 1435, 1375, 1260, 1065, 830, 740.

PMR ($CDCl_3$, 100 MHz) δ ppm: 1.59 (q, 2H, J=8 Hz, $-CH_2CH_2CHOH$), 1.64, 1.71 (each s, each 3H, $-CH_3$x2), 2.15 (bq, 2H, J=8 Hz, $\geq C=CHCH_2-$), 3.00 (bs, 1H, -OH), 3.30-4.00 (m, 3H, $HOCHCH_2Cl$), 5.14 (bt, 1H, J=8 Hz, $\geq C=CH$).

(B) 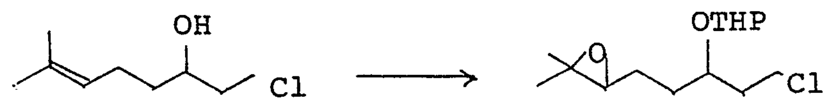

The chlorohydrin compound (1-chloro-6-methyl-5-hepten-2-ol, 600 mg, 90% purity, net content: 540 mg, 3.32 mmole) and 2,3-dihydropyran (370 mg, 4.42 mmole) were dissolved in ethyl acetate (0.6 ml) and several milligrams of p-toluenesulfonic acid was added thereto. After the exotherm abated, sufficient triethylamine was added to neutralize the reaction mixture. The reaction mixture was then concentrated at reduced pressure and the resulting residue was dissolved in methylene chloride (7 ml). After addition of sodium bicarbonate (535 mg), m-chloroperbenzoic acid (80% purity, 749 mg, 3.49 mmole) was added in portions with stirring under cooling on an ice-water bath and stirred for 30 minutes. After disappearance of the starting material was confirmed, saturated sodium sulfite solution (1 ml) and water (2 ml) were added and stirred for 1 hour. The organic layer was separated and the aqueous layer was extracted with methylene chloride. The organic layer and the extract were combined and washed with water, dried and concentrated. The residue was subjected to chromatography on silica gel column and eluted with n-hexane-ethyl acetate (4:1). The appropriate fractions were concentrated to give the desired epoxy compound (1-chloro-6-methyl-5,6-epoxy-2-heptyl-tetrahydropyran-2-yl-ether, 850 mg, 97.5%).

IR (film) $cm^{-1}$ : 2950, 2860, 1375, 1200, 1130, 1075, 1035, 1025, 985, 905, 870.

PMR ($CDCl_3$, 100 MHz) δ ppm: 1.34 (each s, each 3H, $CH_3$x2), 2.76 (bm, 1H, 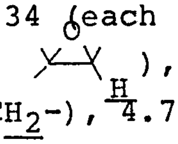), 3.3-4.1 (m, 5H, $-OCHCH_2-$, $-OCH_2-$), 4.72 (bm, 1H, -OCHO-).

(C)

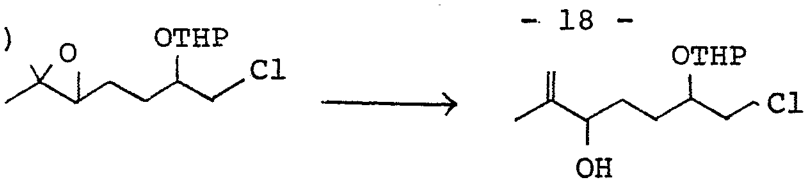

The epoxy compound (1-chloro-5,6-epoxy-6-methyl-2-heptyl-tetrahydropyran-2-yl-ether, 1.45 g, 5.52 mmole) was dissolved in toluene (10 ml) and aluminumtriisopropoxide (1.18 g, 5.8 mmole) was added thereto and heated at reflux for 6 hours. After cooling, 2N hydrochloric acid (3 ml) was added and stirred for 1 hour and the organic layer was separated. The organic layer was washed successfully with water, saturated sodium bicarbonate solution and water, dried ($MgSO_4$) and concentrated. The residue was purified by subjecting to chromatography on silica gel column and eluting with n-hexane-ethyl acetate (4:1) to give the desired allyl alcohol compound (1-chloro-5-hydroxy-6-methylene-2-heptyl-tetrahydropyran-2-yl-ether, 1.35 g, 5.14 mmole).

IR (film) $cm^{-1}$ : 3400, 3070, 2940, 2860, 1645, 1440, 1130, 1075, 1020, 980, 900, 870.

PMR ($CDCl_3$, 100 MHz) $\delta$ ppm: 1.72 (s, 3H, $CH_3$), 2.90 (bm, 1H, -OH), 4.75 (m, 1H, -OCHO-), 4.84 and 4.95 (each s, each 1H, $H_2C=C<$).

(D)

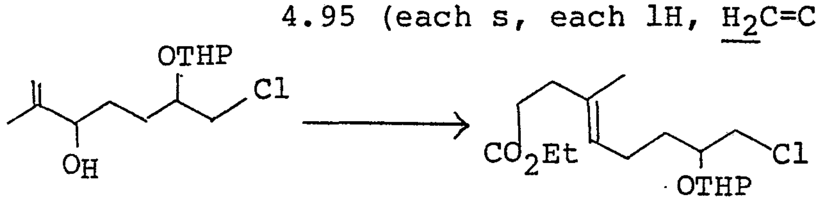

The allyl alcohol compound (1-chloro-5-hydroxy-6-methylene-2-heptyl-tetrahydropyran-2-yl-ether, 1.14 g, 4.34 mmole) was dissolved in triethyl orthoacetate (4.2 g, 26 mmole) and a drop of propionic acid was added thereto. The mixture was heated on an oil bath at 140°C while the ethanol formed by the reaction was distilled off. After 1 hour, the excess triethyl orthoacetate and propionic acid were distilled off at reduced pressure and the residue was purified by subjecting chromatography on a silica gel column and eluting with n-hexane-ethyl acetate (7:1) to give the desired ester (1-chloro-6-carbethoxyethyl-5-hepten-2-yl-tetrahydropyran-2-yl-ether, 1.31 g, 4.30 mmole, 99%).

IR (film) $cm^{-1}$ : 2930, 2860, 1730, 1445, 1370, 1340, 1260, 1075, 1030, 990, 870.

PMR ($CDCl_3$, 100 MHz) $\delta$ ppm: 1.24 (t, 3H, J=6 Hz, $CH_3CH_2-$), 1.63 (s, 3H, $CH_3C=C<$), 4.16 (q, 2H, J=6 Hz, $CH_3CH_2-$), 4.64 and 4.76 (each bs, total 1H, -OCHO-), 5.20 (bm, 1H, $CH=C<$).

(E)

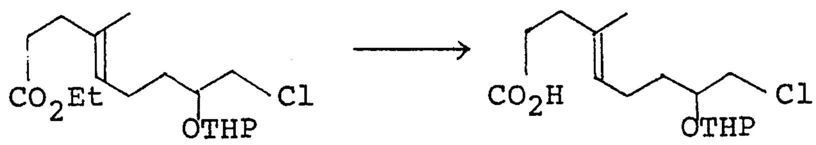

A mixture of the ester (1-chloro-6-carbethoxyethyl-5-hepten-2-yl-tetrahydropyran-2-yl-ether, 1.0 g, 3.28 mmole), 2N sodium hydroxide solution (2.5 ml) and methanol (5 ml) was allowed to react under stirring at room temperature for 4 hours. Most of the methanol was then distilled off at reduced pressure from the reaction mixture. Thereafter water (5 ml) was added and the mixture was washed with n-hexane and acidified with 6N hydrochloric acid (to pH 1). The organics were then extracted with ethyl acetate and the ethyl acetate layer was washed with water, dried and concentrated to give a carboxylic acid (1-chloro-6-carboxyethyl-5-hepten-2-yl-tetrahydropyran-2-yl-ether, 940 mg, 94%).

IR (film) $cm^{-1}$ : 3100, 2950, 2870, 2670, 1710, 1405, 1135, 1120, 1080, 1025.

PMR ($CDCl_3$, 100 MHz) $\delta$ ppm : 1.64 (s, 3H, $CH_3$-), 3.40-4.10 (m, 5H, $ClCH_2C\underline{H}O$-, -$\underline{CH_2}O$-), 4.68 and 4.80 (each bm, total 1H, -OC$\underline{H}$O-), 5.20 (bt, 1H, J=7 Hz, $\overset{1}{C}\underline{H}$=C<), 10.5 (bs, 1H, O$\underline{H}$).

(F)

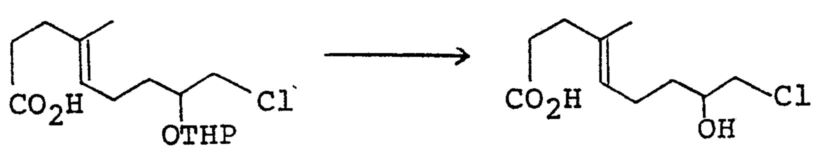

The pyranyl-ether compound (1-chloro-6-carboxyethyl-5-hepten-2-yl-tetrahydropyran-2-yl-ether, 9.40 g, 3.09 mmole) was dissolved in a mixture of tetrahydrofuran (90 ml) and 2N hydrochloric acid (30 ml). After the resulting solution was allowed to stand for 24 hours, most of the tetrahydrofuran was distilled off at reduced pressure. The residue was extracted with ethyl acetate and the separated organic layer was washed with water, dried and concentrated. The residue was purified by silica gel column chromatography [eluting with n-hexane-ethyl acetate (2:1)] to give the desired chlorohydrin compound (9-chloro-8-hydroxy-4-methyl-4-nonenoic acid, 6.40 g,

94%).

IR (film) $cm^{-1}$ : 3350, 2910, 2640, 1705, 1440, 1265, 1200, 1160, 1100, 1060, 910, 735.

PMR ($CDCl_3$, 100 MHz) δ ppm : 1.67 (s, 3H, $-CH_3$), 2.00-2.60 (m, 8H, $-(CH_2)_2-$x2), 3.35-3.95 (3H, m, $-C\underline{H}OHCH_2Cl$), 5.19 (bt, 1H, J=7 Hz, $-C\underline{H}=C<$), 6.80 (bs, 1H, $-O\underline{H}$).

<u>Example 3</u>

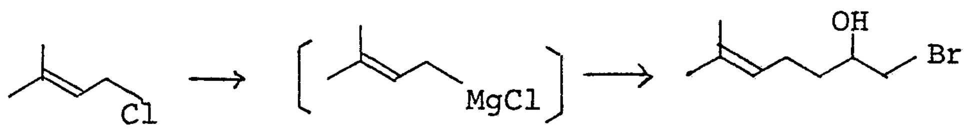

Mangesium (15.4 g, 0.634 mole) was added to tetrahydrofuran (600 ml) and the mixture was cooled to -10°C. To the cooled mixture was added dropwise a solution of prenyl chloride (60 g, 0.574 mole) in tetrahydrofuran (300 ml) over 2 hours and 30 minutes under stirring. After stirring for 30 minutes to allow the mixture to react, the reaction mixture was added dropwise to a suspension of epibromohydrin (79 g, 0.581 mole) and cuprous iodide (11 g, 57.8 mmole) in tetrahydrofuran (200 ml) under adequate stirring at such a rate that the reaction temperature was kept at -10°C or below. After completion of the addition, stirring was continued for an additional 30 minutes and acetic acid (35 g, 0.583 mole) was then added. The tetrahydrofuran was distilled off at reduced pressure and to the residue were added saturated ammonium chloride solution (200 ml) and water (100 ml) and stirred for 4 hours. The organic layer was separated and the aqueous layer was extracted with ethyl acetate (100 ml) three times. The organic layer and the ethyl acetate extracts were combined and dried, concentrated and distilled in vacuo to give 1-bromo-6-methyl-5-hepten-2-ol (93.6 g, 64% purity, 51% net yield), b.p. 60 - 64°C/0.5 mmHg.

IR (film) $cm^{-1}$ : 3300, 2960, 2910, 2850, 1670, 1440, 1375, 1080, 1050, 1010, 830.

PMR ($CDCl_3$, 100 MHz) δ ppm : 1.60 (q, 2H, J=6.5 Hz, $HOCHC\underline{H_2}CH_2-$), 1.64 and 1.71 (each s, each 3H, $C\underline{H_3}$x2), 2.13 (bq, 2H, J=6.5 Hz, $>C=CHC\underline{H_2}-$), 2.21 (bs, 1H, $O\underline{H}$), 3.37 (dd, 1H, J=15 and 6.5 Hz, $-C\underline{Ha}HbBr$), 3.50 (dd, 1H, J=15 and 3.5 Hz, $-CHa\underline{Hb}Br$), 3.75 (dq,

1H, J=6.5 and 3.5 Hz, -CHOH), 5.07 (bt, 1H, J=6.5 Hz, CH=C<).

Example 4

A chlorohydrin compound (1-chloro-6-methyl-5-hepten-2-ol, 1.5 g, 9.23 mmole, 96% purity, net 8.86 mmole) as prepared in Example 2(A) and ethyl vinyl ether (798 mg, 11 mmole) were dissolved in ethyl acetate (1.5 ml) and p-toluenesulfonic acid (5 mg) was then dissolved therein, thereby causing exotherm to initiate. After about 15 minutes, disappearance of the starting material was confirmed and thereafter the reaction mixture was neutralized with triethylamine and then concentrated. The residue was subjected to chromatography on silica gel column and eluted with n-hexane-ethyl acetate (15:1). The appropriate fractions were collected and concentrated to give 1-chloro-6-methyl-5-hepten-2-yl-(1-ethoxyethyl)-ether (2.06 g, 99%).

IR (film) $cm^{-1}$ : 2970, 2920, 1670, 1445, 1375, 1125, 1095, 1055, 950, 745.

PMR ($CDCl_3$, 100 MHz) $\delta$ ppm : 1.21 (t, 3H, J=6.5 Hz, $CH_3CH_2$-), 1.35 and 1.36 (each d, total 3H, J=5 and 6 Hz, $CH_3CH<^{O-}_{O-}$), 1.64 and 1.72 (each s, each 3H, $(CH_3)_2C$=C<), 1.88-2.24 (m, 2H, $-CH_2$-C=C<), 3.35-4.00 (m, 5H, $-OCH_2CH_3$, $-OCHCH_2Cl$), 2.76 and 2.85 (each q, total 1H, J=6 and 5 Hz, -OCHO-), 5.12 (m, 1H, -CHC=C<).

Example 5

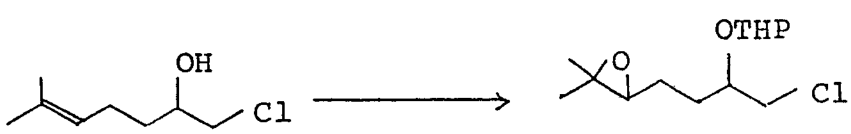

An olefin compound (1-chloro-6-methyl-5-hepten-2-ol, 96% purity, 1.5 g, 8.86 mmole) as prepared in Example 2(A) and 2,3-dihydropyran (853 mg, 10.2 mmole) was dissolved in ethyl acetate (1.5 ml) and several milligrams of p-toluenesulfonic acid was added. The mixture was allowed to react for 30 minutes, whereupon disappearance of the starting compound was confirmed. Thereafter, a sufficient amount of triethylamine was added to neutralize the reaction mixture. The ethyl acetate was then distilled off. The residue was dissolved in tetrahydrofuran (13 ml) and water (3.3 ml) was added to the solution. To the

resulting mixture cooled on an ice-water bath was added dropwise N-bromosuccinimide (1.65 g, 9.3 mmole) over about 10 minutes under thorough stirring. The mixture was further stirred for another 30 minutes under the same conditions to yield a homogeneous pale yellow solution. A solution of sodium hydroxide (500 mg) in methanol (5 ml) was then added dropwise to the pale yellow solution and the mixture was allowed to react for 1 hour at room temperature under stirring. Most of the tetrahydrofuran was distilled off from the reaction mixture at reduced pressure and ethyl ether was added to the remaining heterogeneous solution. The organic layer was separated and it was washed with water and saturated saline, dried ($MgSO_4$) and concentrated. The residue was subjected to chromatography on silica gel column (eluting with a 5:1 mixture of n-hexane and ethyl acetae) to give the desired epoxy compound (1-chloro-5,6-epoxy-6-methylheptan-2-yl-tetrahydropyran-2-yl-ether, 1.74 g, 75%).

<u>Example 6</u>

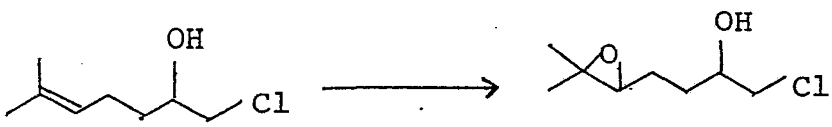

To a mixture of an olefin compound (1-chloro-6-methyl-5-hepten-2-ol, 1.5 g, 9.23 mmole) as prepared in Example 2(A) and sodium bicarbonate (1.55 g) in methylene chloride (25 ml) was added dropwise m-chloroperbenzoic acid (2.08 g, 9.69 mmole) over 10 minutes on an ice-water bath under thorough stirring. After completion of the addition, the mixture was stirred for 1 hour. Thereafter, saturated sodium sulfite solution (5 ml) and water (10 ml) were added and stirring was continued for another hour. The methylene chloride layer was separated from the aqueous layer, washed with water and saturated saline, dried ($MgSO_4$) and concentrated to give a stoichiometric yield of the epoxy compound (1-chloro-5,6-epoxy-6-methyl-heptan-2-ol, 1.64 g).

IR (film) $cm^{-1}$ : 3400, 2960, 2920, 2870, 1460, 1425, 1380, 1320, 1250, 1120, 1080, 1060, 1035, 1020, 745.

PMR ($CDCl_3$, 100 MHz) δ ppm : 1.30, 1.34 (each s, each 3H, $CH_3$x2), 1.4-2.0 (m, 4H, $-\underline{CH_2}\underline{CH_2}-$), 2.78 (bt, 1H, J=5.5 Hz, 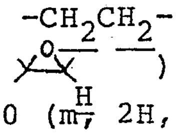), 3.05 (bs, 1H, -O<u>H</u>), 3.50-3.70 (m, 2H, $-\underline{CH_2}Cl$),

3.86 (bm, 1H, -CHOH)

Example 7

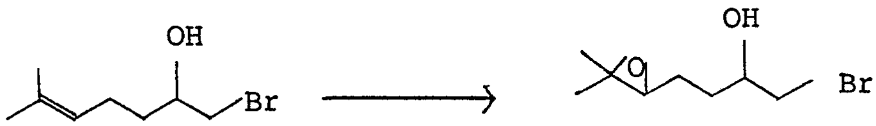

An olefin compound (1-bromo-6-methyl-5-hepten-2-ol, 515 mg, 2.5 mmole) as prepared in Example 3 was dissolved in methylene chloride (10 ml) and m-chloroperbenzoic acid (80% purity, 591 mg, 2.75 mmole) was then gradually added thereto with stirring under ice cooling. After completion of the addition, stirring was continued for 30 minutes and to the reaction mixture were added saturated sodium sulfite solution (5 ml), saturated sodium bicarbonate solution (5 ml) and water (5 ml). After stirring for an additional 30 minutes, the organic layer was separated from the aqueous layer, washed with water, dried ($MgSO_4$) and concentrated. The residue was purified by silica gel column chromatography [eluting with n-hexane-ethyl acetate (5:1)] to give the desired epoxy compound (1-bromo-6-methyl-5,6-epoxy-2-heptanol, 516 mg, 93%).

IR (film) $cm^{-1}$ : 3300, 2970, 2930, 2870, 1454, 1435, 1375, 1220, 1155, 1165, 980, 960.

PMR ($CDCl_3$, 100 MHz) $\delta$ ppm : 1.28, 1.32 (each s, each 3H, $-CH_3x2$), 1.40-2.00 (m, 4H, $-CH_2CH_2-$), 2.76 (bt, 1H, J=5.5 Hz, 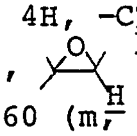 ), 2.78 (bs, 1H, -OH), 3.20-3.60 (m, 2H, $-CH_2Br$), 3.81 (bm, 1H, -CHOH-).

Having now fully described the invention, it will be apparent to one of ordinary skill in the art that many changes and modifications can be made thereto without departing from the spirit or scope of the invention as set forth herein.

WHAT IS CLAIMED AS NEW AND INTENDED TO BE COVERED BY LETTERS PATENT IS:

1. A 9-halo-8-hydroxy-4-methyl-4-nonenoic acid and its derivative of the formula:

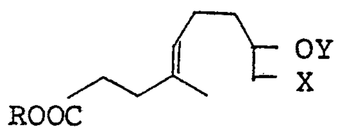

(I)

wherein X is a halogen atom, Y is hydrogen or a hydroxy-protecting group, and R is hydrogen or a lower alkyl.

2. A process for preparing a 9-halo-8-hydroxy-4-methyl-4-nonenoic acid or its derivative of the formula:

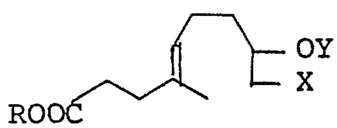

(I)

wherein X is a halogen atom, Y is hydrogen or a hydroxy-protecting group, and R is hydrogen or a lower alkyl,

which comprises reacting a prenyl halide of the formula:

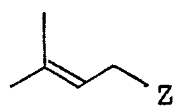

(II)

wherein Z is a halogen atom,

with magnesium to give a prenyl magnesium halide, then reacting the resulting compound with an epihalohydrin of the formula:

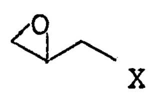

(III)

wherein X is as defined above,

and, if desired, protecting the hydroxyl group of the product with a protecting agent to give a 1-halo-6-methyl-5-hepten-2-ol or its derivative of the formula:

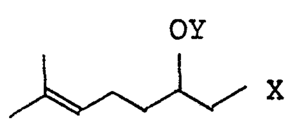

(IV)

wherein X and Y are as defined above,

then epoxidizing the resulting compound to give a 1-halo-5,6-epoxy-6-methyl-2-heptanol or its derivative of the formula:

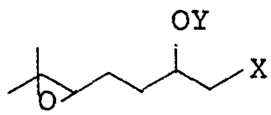

(V)

wherein X and Y are as defined above, subjecting the resulting epoxy compound to rearrangement to give a 1-halo-5-hydroxy-6-methylene-2-heptanol or its derivative of the formula:

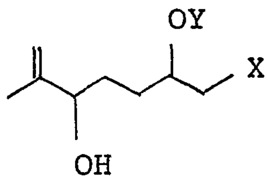

(VI)

wherein X and Y are as defined above, reacting the rearranged product with an orthoacetic acid ester of the formula:

$$CH_3C(OR')_3 \qquad (VII)$$

wherein R' is a lower alkyl, in the presence of a weak acid, followed by hydrolysis, to give the desired 9-halo-8-hydroxy-4-methyl-4-nonenoic acid or its derivative.

3. A 1-halo-6-methyl-5-hepten-2-ol and its derivative of the formula:

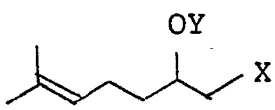

(IV)

wherein X is a halogen atom and Y is hydrogen or a hydroxy-protecting group.

4. A 1-halo-5,6-epoxy-6-methyl-2-heptanol and its derivative of the formula:

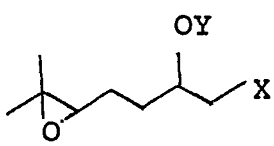

(V)

wherein X is a halogen atom and Y is hydrogen or hydroxy-protecting group.

5. A 1-halo-5-hydroxy-6-methylene-2-heptanol and its derivative of the formula:

0054909

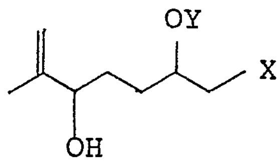

(VI)

wherein X is a halogen atom and Y is hydrogen or hydroxy-protecting group.